# EUROPÄISCHE PATENTSCHRIFT

(11) **EP 0 254 100 B1**
(45) Veröffentlichungstag und Bekanntmachung des Hinweises auf die Patenterteilung: **15.06.1994**
(21) Anmeldenummer: 87109568.3
(22) Anmeldetag: 03.07.1987
(51) Int. Cl.: A61M 5/14

(54) **Vorrichtung zum Abtrennen von Gasblasen aus Flüssigkeiten**
Apparatus for eliminating gas bubbles from liquids
Dispositif pour éliminer les bulles de gaz des fluides

(30) Priorität: 18.07.1986 DE 3624363
(43) Veröffentlichungstag der Anmeldung: 27.01.1988
(73) Patentinhaber: Akzo Nobel N.V., 6824 BM Arnhem (NL)
(72) Erfinder: Baurmeister, Ulrich, Dr., D-5600 Wuppertal 1 (DE); Pelger, Michael, Dr., D-5600 Wuppertal 22 (DE)
(74) Vertreter: Fett, Günter

(56) Entgegenhaltungen:
- EP-A- 0 014 403
- EP-A- 0 138 060
- DE-A- 1 949 038
- DE-A- 1 959 679
- DE-A- 2 737 745
- FR-A- 2 183 113
- GB-A- 1 301 015
- GB-A- 2 092 153
- GB-A- 2 134 812
- US-A- 4 276 170

## Beschreibung

Die Erfindung betrifft eine Vorrichtung zum Abtrennen von Gasblasen aus Infusionsflüssigkeiten oder Flüssigkeiten des menschlichen Körpers mittels mindestens einem flüssigkeitsabweisenden gasdurchlässigen mikroporösen Entlüftungselement mit einem Gehäuse, welches eine Einlaßöffnung für die zu entgasende Flüssigkeit, eine Auslaßöffnung für die entgaste Flüssigkeit und mindestens eine Auslaßöffnung für Gas aufweist, wobei das Entlüftungselement zumindest teilweise in dem Bereich des Gehäuses angeordnet ist, in dem die Gasblasen auftreten.

Derartige Vorrichtungen sind mehrfach bekannt (DE-A-19 59 679, DE-C-23 17 750, DE-A-33 04 951). Bei den bekannten Vorrichtungen ist das Entlüftungselement eine Flachmembran (DE-A-19 59 679, DE-C-23 17 750) oder zumindest ein Teil des Gehäuses (DE-A-33 04 951). Diese Vorrichtungen werden in der Regel am Arm des Patienten befestigt, wodurch zwar das Gehäuse bezüglich der Längsachse nicht mehr verdreht, jedoch durch die Armbewegung des Patienten in Schräglage gebracht werden kann. Insofern muß das Entlüftungselement in Längsrichtung des Gehäuses gesehen an beiden Enden und im Bereich oder oberhalb der Längsachse des Gehäuses angeordnet sein, um bei jeder Schräglage des Gehäuses eine sichere Abtrennung der Gasblasen aus der Flüssigkeit zu erreichen. Bei der Verwendung von Flachmembranen als Entlüftungselement ist erhöhter Arbeitsaufwand zur dichten Einbettung der Entlüftungselemente bei der Herstellung erforderlich, wodurch die bekannten Vorrichtungen teuer sind, was sich besonders deshalb nachteilig auswirkt, da diese Vorrichtungen Wegwerfartikel sind und deshalb als Massenartikel produziert werden. Da diese Vorrichtungen in der Regel mit Heftpflaster auf dem Arm des Patienten befestigt werden, besteht die Gefahr, daß die Auslaßöffnung für das Gas, die häufig als Öffnung in der dem Patientenarm abgewandten Gehäusewand ausgebildet ist, durch das Heftpflaster abgedeckt wird, so daß eine wirksame Entlüftung der Flüssigkeit unterbunden wird.

Aus DE-A-19 49 038 ist ferner eine Vorrichtung zum Abtrennen von Gasblasen aus Flüssigkeiten gemäß Oberbegriff von Anspruch 1 bekannt, bei der ein rohrförmiges, am einen Ende abgeschlossenes Element in der Ausgangskappe angeordnet ist, bei dem die Seitenwand flüssigkeitsabweisend und gasdurchlässig ist und der das Ende abschließende Teil als Filter wirkt. Die durch die Seitenwand austretende Luft kann durch Löcher in der Seitenwand der Ausgangskappe entweichen. Alle bekannten Vorrichtungen weisen insgesamt den Nachteil auf, daß aufgrund der geforderten Mikroporosität der Entlüftungselemente diese nicht durchsichtig sind und insofern nicht kontrolliert werden kann, ob die Gasblasen aus der Flüssigkeit entfernt werden.

Aufgabe der vorliegenden Erfindung ist es, eine gattungsgemäße Vorrichtung zum Abtrennen von Gasblasen aus Flüssigkeiten zur Verfügung zu stellen, bei der die Wirksamkeit des Entlüftungselementes soll auch bei ungünstiger Anbringung des Heftpflasters gewährleistet ist.

Die erfindungsgemäß gestellten Aufgaben werden dadurch gelöst, daß das Entlüftungselement aus mindestens einer Entlüftungskapillaren besteht, deren Innenhohlraum gegenüber der Flüssigkeit abgeschlossen ist, wobei zumindest ein Ende jeder Entlüftungskapillare als Auslaßöffnung für das Gas dient und in eine Dichtmasse eingebettet ist und jede Auslaßöffnung für das Gas mit einer von der Ausgangskappe der Dichtmesser und dem Gehäuse gebildeten umlaufenden Nut oder mit einer von der Ausgangskappe und einem in das Gehäuse eingesetzten Ring gebildeten umlaufenden Nut in Verbindung steht.

Das Gehäuse kann bevorzugt aus transparentem Material hergestellt werden, wodurch die Kapillare(n), deren Wand aus einem flüssigkeitsabweisenden gasdurchlässigen mikroporösen Material besteht, und die Existenz von Gasblasen in der Regel gut beobachtet werden kann. Es versteht sich von selbst, daß Lage und Länge der Entlüftungskapillare(n) derart gewählt sind, daß die Entlüftungsfunktion gewährleistet ist, d.h. die Entlüftungskapillare(n) zumindest in dem Teil des Gehäuses angeordnet ist (sind), in dem Gasblasen auftreten.

Auf einem anderen technischen Gebiet wurden zwar schon die Eigenschaften gasdurchlässiger Hohlfäden ausgenutzt (EP-A-138 060). Bei der bekannten Vorrichtung ist keine Möglichkeit vorgesehen, Gas getrennt aus der Vorrichtung abzuführen. Diese Vorrichtung ist also nicht für das Abtrennen von Gasblasen aus Infusionsflüssigkeiten oder Flüssigkeiten des menschlichen Körpers geeignet.

Im einfachsten Fall kann sich die Entlüftungskapillare über die gesamte oder nahezu gesamte Länge des Gehäuses erstrecken, wobei bevorzugt zumindest jeweils ein in axialer Richtung des Gehäuses angeordnetes Ende der Kapillare(n) als Auslaßöffnungen für das Gas dient. Es versteht sich von selbst, daß entweder beide Enden der Entlüftungskapillaren Austrittsöffnungen sind oder das nicht als Austrittsöffnung verwendete Ende gegenüber der Flüssigkeit verschlossen ist.

Geeignete Werkstoffe für die flüssigkeitsabweisenden, gasdurchlässigen mikroporösen Entlüftungskapillaren sind beispielsweise Polypropylen, Polyvinylidenfluorid oder Polytetrafluoräthylen. Die Herstellung solcher Entlüftungskapillaren kann beispielsweise nach den in der DE-A-30 49 557 oder DE-A-27 37 745 beschriebenen Verfahren erfolgen. Eine besonders günstige Ausführungsform der erfindungsgemäßen Vorrichtung ergibt sich dann, wenn diese bei Verdrehung um die Längsachse in jeder (Dreh-)Lage eine gute Entlüftungsfunktion aufweist. Dies wird bei der erfindungsgemäßen Vorrichtung dadurch erreicht, daß mehrere Entlüftungskapillaren am Umfang auf der Innenseite des Gehäusemantels verteilt angeordnet sind.

Es ist von Vorteil, wenn mehrere parallel zueinander angeordnete Entlüftungskapillaren in Form eines Bändchens eingesetzt werden. Diese Kapillaren können entweder in Strömungsrichtung oder in Strömungsrichtung und quer zur Strömungsrichtung angeordnet sein. Das Bändchen kann auch Schlauchform aufweisen, welche Schlauchform einseitig - beispielsweise in der Art eines Fingerhuts - geschlossen ausgebildet ist. Die Form eines Bändchens läßt sich auf einfache Weise auch dadurch erreichen, daß die Entlüftungskapillaren Schuß und/oder Kettfäden eines Gewebes sind. Sofern nur die Schuß- oder nur die Kettfäden Entlüftungskapillaren sind, werden für die andere Fadensorte (Kett- bzw. Schußfäden) Garne oder Fäden eingesetzt, die lediglich mit der zu entgasenden Flüssigkeit verträglich sein müssen. In der Regel werden hierzu Fäden aus synthetischen Polymeren verwendet.

In der erfindungsgemäßen Vorrichtung kann zusätzlich ein benetzbares gasundurchlässiges mikroporöses Filter enthalten sein, welches das Gehäuse in eine erste Kammer mit der Einlaßöffnung und in eine zweite Kammer zusammen mit der Ausgangskappe trennt, wobei die Entlüftungskapillare(n) in der ersten Kammer zwischen Filter und Gehäusewand zumindest oberhalb des Filters angeordnet ist (sind).

Wenn hier davon die Rede ist, daß die Entlüftungskapillare oberhalb des Filters angeordnet ist, so soll dies zum Ausdruck bringen, daß die Entlüftungskapillare bei Arbeitslage im Gehäuse oberhalb des Filters angeordnet ist.

Zum Filtern der Flüssigkeit geeignete Filter, die auch Membranen sein können, können die Eigenschaft, durch die zu filternde Flüssigkeit benetzbar, also beispielsweise lyophil oder hydrophil, zu sein, von Natur aus besitzen oder aber erst durch eine spezielle Behandlung erhalten haben. Dasselbe gilt sinngemäß für den flüssigkeitsabweisenden, beispielsweise lyophoben oder hydrophoben, gasdurchlässigen Werkstoff für die Entlüftungskapillare.

Geeignete Werkstoffe der Filtermembran sind beispielsweise Polyamid 6.6, Polyamid 6, Cellulose, Celluloseacetat oder Polyvinylalkohol (PVA). Geeignete Werkstoffe für die flüssigkeitsabweisenden gasdurchlässigen mikroporösen Teile der erfindungsgemäßen Vorrichtung sind beispielsweise Polypropylen, Polyvinylidenfluorid oder Polytetrafluoräthylen.

Die mittlere Porengröße für das Filter richtet sich nach dem jeweiligen gewünschten Filtereffekt und beträgt beispielsweise 0,2 »m zur Sterilfiltration oder 1 »m für Partikelfiltration.

Die maximale Porengröße des benetzbaren gasundurchlässigen mikroporösen Filters (Membran) muß jedoch so beschaffen sein, daß der zugehörige Blasdruck (d.h. der Druck, bei dem Gas durch die Filterschicht dringt, indem es die Flüssigkeit aus den Poren verdrängt) größer ist als der Filtrationsdruck. Der Blasdruck und damit der anwendbare Filtrationsdruck kann umso größer sein, je kleiner die maximale Pore der benetzbaren mikroporösen Filterschicht ist.

Andererseits muß die Porengröße der flüssigkeitsabweisenden gasdurchlässigen mikroporösen Entlüftungskapillare so gewählt werden, daß der Filtrationsdruck kleiner ist als der für die Flüssigkeitsintrusion notwendige Druck. Der Druck für die Flüssigkeitsintrusion, der auch als Flüssigkeitseindringdruck bezeichnet wird, ist umso größer, je kleiner die maximale Porengröße der Entlüftungskapillare ist und je größer die Differenz der Oberflächenspannungen von Flüssigkeit und dem flüssigkeitsabweisenden gasdurchlässigen mikroporösen Werkstoff ist.

Diese für die Wechselwirkung von Flüssigkeiten und Gasen mit mikroporösen Werkstoffen bestehenden Beziehungen sind bei der Konstruktion und dem Gebraucch der erfindungsgemäßen Vorrichtung zu beachten.

Die Abtrennung von Gasen aus Flüssigkeiten mittels mikroporösen flüssigkeitsabweisenden Werkstoffen ist dann kein Problem, wenn es sich um Flüssigkeiten mit einer großen Oberflächenspannung handelt, wie z.B. Wasser.

Eine Verringerung der Differenz der Oberflächenspannung von Flüssigkeit und Werkstoff, z.B. bei Anwesenheit von die Oberflächenspannung herabsetzenden Additiven, kann man durch Reduzierung der Porengröße des Werkstoffes bzw. durch Auswahl eines Werkstoffes mit geeigneter Porengröße kompensieren. Gegebenenfalls muß dann die für die Entgasung zur Verfügung stehende Oberfläche vergrößert werden. Darüber hinaus kann die flüssigkeitsabweisende Eigenschaft eines Werkstoffes auch bei hohen Flüssigkeitstemperaturen beeinträchtigt werden oder gänzlich verloren gehen. Das bedeutet, daß für die das Abtrennen von Gasen aus der Flüssigkeit bewirkenden porösen Teile der erfindungsgemäßen Vorrichtung ein Werkstoff zu wählen ist, mit dem unter den gegebenen Betriebsbedingungen das Austreten von Flüssigkeit verhindert wird. Durch einen einfachen Vorversuch läßt sich ermitteln, ob die bei Normalbedingungen gegebene flüssigkeitsabweisende Eigenschaft eines porösen Werkstoffes auch unter Betriebsbedingungen noch gegeben ist.

Die erfindungsgemäße Vorrichtung ist auch dann geeignet, wenn zu ihrem bestimmungsgemäßen Gebrauch in der Flüssigkeit ein nennenswerter Überdruck gegenüber dem Umgebungsdruck vorherrscht, wie dies beispielsweise bei Infusionsfiltern mit vorgeschalteter Pumpe der Fall sein kann.

Besonders vorteilhaft ist es, wenn das Filter aus mindestens einer Filterkapillaren besteht, und der Innenhohlraum der Filterkapillaren zumindest teilweise die zweite Kammer bildet. Gegenüber Flachfiltern wird über Filterkapillare eine besonders große wirksame Filteroberfläche erreicht.

Die Entlüftung der Flüssigkeit gelingt dann vorzüglich, wenn die Entlüftungskapillare(n) die Filterkapillare(n) über ihre Länge mehrfach locker umschlingend angeordnet ist (sind).

Es ist auch möglich, daß bei der erfindungsgemäßen Vorrichtung die Filterkapillaren in Form eines Bündels angeordnet sind, an dessen Umfang die Entlüftungskapillaren angeordnet sind.

Besonders dann, wenn die Innenhohlräume der Entlüftungskapillaren im Bereich der Einlaßöffnung der zu entgasenden Flüssigkeit mit der umliegenden Atmosphäre Kontakt haben, können Entlüftungskapillaren und Filterkapillaren in jeder gewünschten Anordnung nebeneinander liegen. Insbesondere kann jede Filterkapillare einer Entlüftungskapillare und jede Entlüftungskapillare einer Filterkapilllare benachbart angeordnet sein.

Bei den Ausführungsformen, bei denen Filterkapillaren und Entlüftungskapillaren nebeneinander angeordnet sein sollen, hat es sich bestens bewährt, daß die Filterkapillaren und die Entlüftungskapillaren als Schuß- und/oder Kettfäden zu einem Gewebe oder mehreren Geweben vereinigt sind. Sofern nur die Schuß- oder nur die Kettfäden Kapillaren sind, werden für die anderen (Kett- bzw. Schuß-)Fäden Garne oder Fäden verwendet, die bevorzugt aus synthetischen Polymeren bestehen, die allerdings mit den zu entgasenden Flüssigkeiten verträglich sein müssen.

Besonders vorteilhaft ist eine Vorrichtung, bei der erfindungsgemäß in einem rohrförmigen Gehäuse die Filterkapilllaren und Entlüftungskapillaren in Form eines langgezogenen U's angeordnet sind, wobei alle Enden der Kapillaren am einen Ende des Gehäuses in eine Dichtmasse derart eingebettet sind, daß im zentralen Bereich der Einbettstelle lediglich Filterkapillaren und im Außenbereich lediglich Entlüftungskapillaren enden, wobei das mit der Dichtmasse versehene Ende des Gehäuses durch eine die Auslaßöffnung für die entgaste Flüssigkeit enthaltende Ausgangskappe derart abgeschlossen ist, daß die Enden der Filterkapillaren innerhalb und die Enden der Entlüftungskapillaren außerhalb der Auslaßöffnung für die entgaste Flüssigkeit enden.

Wenn hier ausgeführt wird, daß die Enden der Kapillaren in eine Dichtmasse eingebettet sind, dann bedeutet dies, daß die Dichtmasse die einzelnen Kapillaren dicht umschließt, die Innenhohlräume der Kapillaren durch die Dichtmasse aber nicht verschlossen sind.

Als rohrförmige Gehäuse kommen Gehäuse mit praktisch allen möglichen Querschnitten infrage, insbesondere mit elliptischem, dreieckförmigem oder kreisförmigem Querschnitt.

Hierbei hat es sich als zweckmäßig erwiesen, wenn die Enden der Entlüftungskapillaren im Bereich der Dichtmasse zumindest annähernd zum Gehäuse achsparallel angeordnet sind.

Um eine gute Abdichtung zwischen Ausgangskappe und Dichtmasse als Trennstelle zwischen den Enden der Filterkapillaren und der Entlüftungskapillaren zu gewährleisten, hat sich ein Ring zwischen Kappenende und Dichtmasse bestens bewährt. Dieser Ring kann in Richtung der Dichtmasse angespitzt sein, so daß der Ring teilweise in die Dichtmasse beim Aufsetzen der Ausgangskappe eindringt. Der Ring kann aber auch vor dem Einbringen der Dichtmasse eingeführt und in dieser eingebettet werden. Er kann mit dem Gehäuse und/oder der Ausgangskappe - beispielsweise durch Kleben - verbunden sein, so daß beispielsweise über den Ring die Ausgangskappe mit dem Gehäuse verbunden ist. Dieser Ring muß nicht unbedingt kreisförmig sein. Polygone oder elliptische Ringe können ebenfalls verwendet werden.

Für die Dichtungsmasse kommen die zum Einbetten von Kapillaren üblichen Werkstoffe, die thermoplastisch oder selbstaushärtend sein können, infrage.

Die Erfindung wird anhand der nachfolgenden Figuren näher erläutert. Es zeigen:
- Figur 1: eine Darstellung eines Anwendungsfalles erfindungsgemäßer Vorrichtungen,
- Figur 2: eine Ausführungsform einer erfindungsgemäßen Entlüftungs- und Filtervorrichtung im Längsschnitt,
- Figur 3: eine weitere Ausführungsform einer erfindungsgemäßen Entlüftungs- und Filtervorrichtung im Längsschnitt.

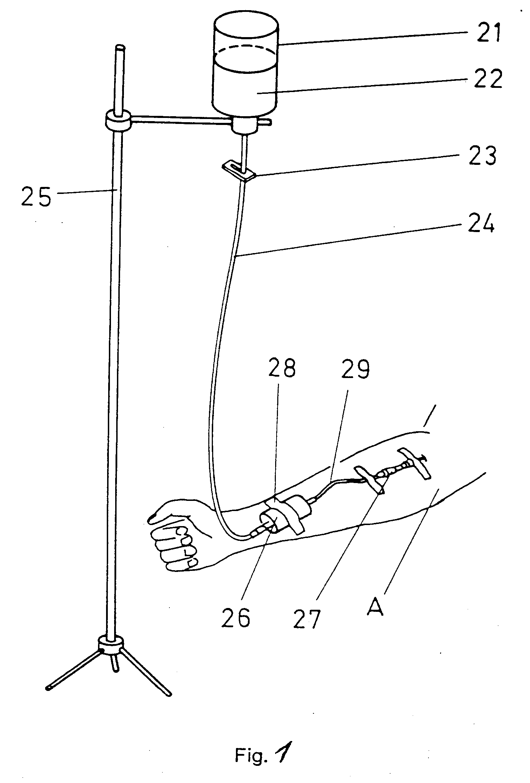

In Figur 1 ist ein Anwendungsfall einer erfindungsgemäßen Vorrichtung 26 dargestellt. Von der Vorratsflasche 21, die in einem Ständer 25 positioniert ist, wird eine Infusionsflüssigkeit 22 über einen Schlauch 24 einer Vorrichtung 26 zum Abtrennen von Gasblasen und Filtern dieser Infusionsflüssigkeit zugeleitet. Diese Entlüftungs- und Filtervorrichtung ist am Unterarm A eines Patienten mit einem Heftpflaster 28 angeklebt. Über die Dosierhilfe 23 kann die Zulaufmenge an Infusionsflüssigkeit reguliert werden. Von der Entlüftungs- und Filtervorrichtung 26 gelangt die Infusionsflüssigkeit über einen weiteren Schlauch 29 und eine Infusionsnadel 27 in die Blutbahn.

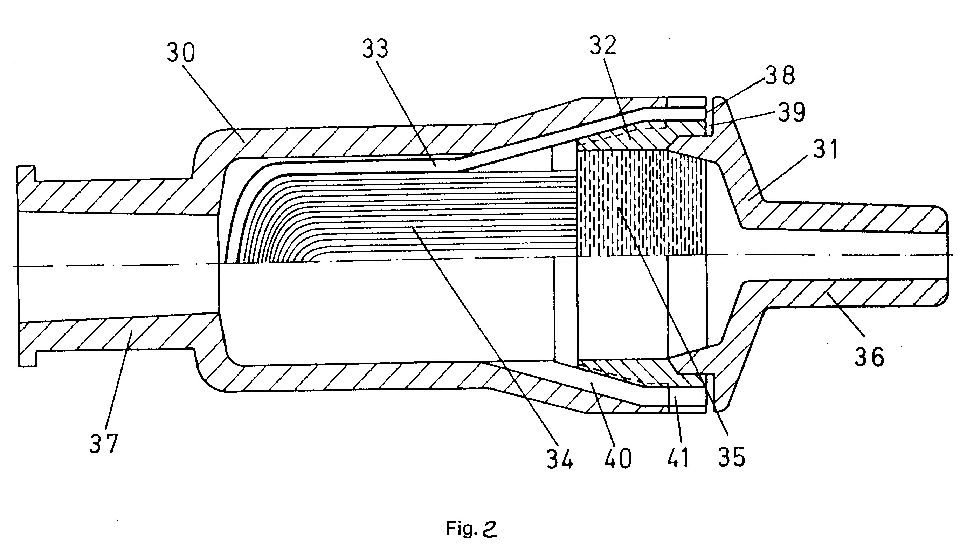

Eine für einen derartigen Anwendungsfall geeignete, erfindungsgemäße Entlüftungs- und Filtervorrichtung ist in Figur 2 dargestellt. Ein rohrförmiges Gehäuse mit einem Einlaßstutzen 37 für die zu entgasende Flüssigkeit ist über einen Ring 32 durch eine Ausgangskappe 31 mit einem Auslaßstutzen 36 abgeschlossen. Im Gehäuse 30 sind mindestens zwei Nuten 40 jeweils paarweise und jeweils diametral gegenüberliegend eingearbeitet, in die die Entlüftungskapillaren 33, die im Gehäuse 30 in Form eines U's liegen, wobei die beiden Enden des U's in jeweils zwei diametral gegenüberliegenden Nuten 40 eingelegt sind, mit Dichtungsmasse eingebettet sind. Der in das Gehäuse eingesetzte Ring 32 schließt die Nuten 40 ab, so daß die Enden der Entlüftungskapillaren 33 im Bereich des Rings 32 dicht eingebettet sind. Weiterhin weist der Ring 32 Nuten oder Bohrungen 41 im äußeren Rand auf, in welche die Entlüftungskapillar-Enden weitergeführt sind oder welche als Weiterführung der Innenhohlräume der Entlüftungskapillaren 33 dienen. Auf der Innenseite des Ringes 32 stützt sich die Ausgangskappe 31 ab, die in nicht dargestellter Weise mit dem Gehäuse beispielsweise über Schnappverschlüsse verbunden ist. Die Ausgangskappe 31 kann aber auch mit dem Ring 32 und/oder der Ring 32 mit dem Gehäuse 30 - beispielsweise über Ultraschallschweißung - verbunden sein. Der Ring 32 dient gleichzeitig zur Aufnahme der Dichtungsmasse 35, in welche Filterkapillaren 34 eingebettet sind, die ebenfalls in Form eines U's in das Gehäuse 30 eingeführt sind. Im Bereich des Gehäuses 30 sind die Filterkapillaren 34 von den Entlüftungskapillaren 33 umgeben, so daß die von den Filterkapillaren 34 abgefilterten Gasblasen in jeder axialen Kipplage durch die Entlüftungskapillaren 33 entweichen können. Werden die Entlüftungskapillaren 33 am Innenumfang des Gehäuses gleichmäßig verteilt, so kann das Gehäuse 30 kreiszylindrischen Querschnitt aufweisen, da nunmehr eine Verdrehung um die Achse des Gehäuses 30 für die Entlüftungsfunktion der Entlüftungskapillaren 33 keine Rolle mehr spielt. Die Enden 38 der Entlüftungskapillaren 33 enden in einer umlaufenden Nut 39, die vom Ring 32 und der Ausgangskappe 31 gebildet wird. Da beim Positionieren der in Figur 2 dargestellten Entlüftungs- und Filtervorrichtung mit Heftpflaster auch dann, wenn das Heftpflaster im Bereich der Nut angeordnet wird, zumindest ein Teil der Nut vom Heftpflaster nicht überklebt ist (z.B. im Bereich zwischen der Entlüftungs- und Filtervorrichtung und dem Patientenarm), ist die Entlüftungsfunktion grundsätzlich gewährleistet.

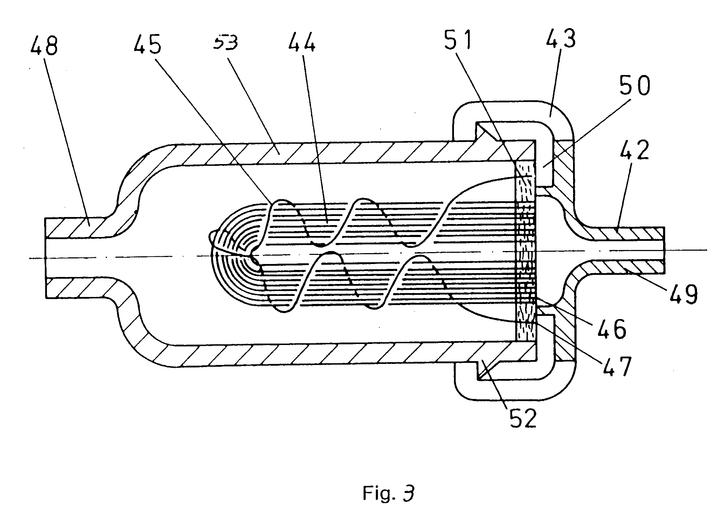

In Figur 3 ist eine weitere vorteilhafte Ausgestaltung einer erfindungsgemäßen Entlüftungs- und Filtervorrichtung dargestellt. Ein Filterkapillaren-Bündel 44, welches in Form eines U's in das Gehäuse 53 eingeführt ist, wird innerhalb des Gehäuses 53 wendelförmig von einer Entlüftungskapillaren 45 umschlungen. Die Enden der Filterkapillaren 44 sind im zentralen Bereich, die Enden der Entlüftungskapillaren 45 am äußeren Umfang in eine Dichtungsmasse 51 eingebettet, wobei die Hohlräume der beiden Kapillararten nach außen offen bleiben. Eine Ausgangskappe 42 ist konstruktiv derart gestaltet, daß sie die Dichtmasse in zwei Bereiche aufteilt, wobei der zentrale Bereich, in welchem die Enden 46 der Filterkapillaren 44 enden, in den Auslaßstutzen 49 der Ausgangskappe 42 mündet, und der äußere Bereich eine von der Ausgangskappe 42, der Dichtmasse 51 und dem Gehäuse 53 gebildete, umlaufende Nut 50 darstellt, in welche die Enden 47 der Entlüftungskapillare 45 enden. Die Ausgangskappe 42 ist über Schnappverschlüsse 43 mit dem Gehäuse 53 verbunden, wobei die Schnappverschlüsse 43 als Bügel ausgebildet und in Nasen 52 des Gehäuses 53 eingerastet sind. Im Bereich der Dichtmasse 51 ist es zweckmäßig, zur besseren Abdichtung zumindest einen Ring vorzusehen (nicht dargestellt). Das Gehäuse 41 weist einen Einlaßstutzen 48 auf.

Bei den in den Figuren 2 und 3 dargestellten Ausführungsformen können die Filterkapillaren 34 und 44 in Form mehrerer, übereinander geschichteter Gewebe vorliegen, wobei die Filterkapillaren 34 oder 44 als Kettfäden in diesem Gewebe liegen. Bei der in Figur 2 dargestellten Ausführungsform können zusätzlich auch die Entlüftungskapillaren 33 als Kettfäden in diesem Gewebe angeordnet sein. Die Entlüftungskapillaren können aber auch lediglich untereinander als Kettfäden verwebt sein. Hierbei empfliehlt es sich besonders, ein Schlauchgewebe zu verwenden.

Wenn bei der in Figur 3 dargestellten Ausführungsform der Schnappverschluß 43 nicht als Steg sondern als umlaufender Ring ausgebildet ist, wird von diesem umlaufenden Ring, von der Ausgangskappe 42, der Dichtmasse 51 und dem Gehäuse 53 ein umlaufender Ringkanal gebildet, in dem durch einen nicht dargestellten Anschlußstutzen im umlaufenden Ring ein Unterdruck angelegt werden kann.

## Patentansprüche

1. Vorrichtung zum Abtrennen von Gasblasen aus Infusionsflüssigkeiten oder Flüssigkeiten des menschlichen Körpers mittels mindestens einem flüssigkeitsabweisenden gasdurchlässigen mikroporösen Entlüftungselement mit einem Gehäuse (30, 53, 31, 42), welches eine Einlaßöffnung (37, 48) für die zu entgasende Flüssigkeit, eine Auslaßöffnung (36, 49) für die entgaste Flüssigkeit in Form einer Ausgangskappe (31, 42) und mindestens eine Auslaßöffnung (38, 47) für das Gas aufweist, wobei das Entlüftungselement zumindest teilweise in dem Bereich des Gehäuses angeordnet ist, in dem Gasblasen auftreten, dadurch gekennzeichnet, daß das Entlüftungselement aus mindestens einer Entlüftungskapillaren (33, 45) besteht, deren Innenhohlraum gegenüber der Flüssigkeit abgeschlossen ist, wobei zumindest ein Ende jeder Entlüftungskapillare (33, 45) als Auslaßöffnung (38, 47) für das Gas dient und in eine Dichtmasse (51) eingebettet ist und jede Auslaßöffnung (38, 47) für das Gas mit einer von der Ausgangskappe (42), der Dichtmasse (51) und dem Gehäuse (53) gebildeten umlaufenden Nut (50) oder mit einer von der Ausgangskappe (31) und einem in das Gehäuse (30) eingesetzten Ring (32) gebildeten umlaufenden Nut (39) in Verbindung steht.

2. Vorrichtung nach Anspruch 1, dadurch gekennzeichnet, daß zumindest jeweils ein in axialer Richtung des Gehäuses angeordnetes Ende (38, 47) der Entlüftungskapillaren (33, 45) als Auslaßöffnung für das Gas dient.

3. Vorrichtung nach Anspruch 1 oder 2, dadurch gekennzeichnet, daß mehrere Entlüftungskapillaren (33) am Umfang auf der Innenseite des Gehäusemantels verteilt angeordnet sind.

4. Vorrichtung nach Anspruch 2 oder 3, dadurch gekennzeichnet, daß mehrere parallel zueinander angeordnete Entlüftungskapillaren in Form eines Bändchens eingesetzt sind.

5. Vorrichtung nach einem oder mehreren der Ansprüche 1 bis 4, dadurch gekennzeichnet, daß diese zusätzlich ein benetzbares gasundurchlässiges mikroporöses Filter (34, 44) enthält, welches das Gehäuse in eine erste Kammer mit der Einlaßöffnung (37, 48) und in eine zweite Kammer zusammen mit der Ausgangskappe (31, 42) trennt, wobei die Entlüftungskapillare(n) (33, 45) in der ersten Kammer zwischen Filter (34, 44) und Gehäusewand zumindest oberhalb des Filters (34, 44) angeordnet ist (sind).

6. Vorrichtung nach Anspruch 5, dadurch gekennzeichnet, daß das Filter aus mindestens einer Filterkapillaren (34, 44) besteht, und der Innenhohlraum der Filterkapillaren zumindest teilweise die zweite Kammer bildet.

7. Vorrichtung nach Anspruch 6, dadurch gekennzeichnet, daß die Entlüftungskapillare(n) (45) die Filterkapillare(n) (44) über ihre Länge mehrfach locker umschlingend angeordnet ist (sind).

8. Vorrichtung nach Anspruch 6, dadurch gekennzeichnet, daß die Filterkapillaren (34) in Form eines Bündels angeordnet sind, an dessen Umfang die Entlüftungskapillare(n) (33) angeordnet ist (sind).

9. Vorrichtung nach Anspruch 6 oder 8, dadurch gekennzeichnet, daß die Filterkapillaren und die Entlüftungskapillaren als Schuß- und/oder Kettfäden zu einem Gewebe oder mehreren Geweben vereinigt sind.

10. Vorrichtung nach einem oder mehreren der Ansprüche 6 bis 9, dadurch gekennzeichnet, daß in einem rohrförmigen Gehäuse die Filterkapillaren (44) und Entlüftungskapillaren (45) in Form eines langgezogenen U's angeordnet sind, wobei alle Enden (46/47) der Kapillaren (44/45) am einen Ende des Gehäuses in eine Dichtmasse (51) derart eingebettet sind, daß im zentralen Bereich der Einbettstelle lediglich Filterkapillaren und im Außenbereich der Einbettstelle lediglich Entlüftungskapillaren enden, wobei das mit der Dichtmasse (51) versehene Ende des Gehäuses durch eine die Auslaßöffnung (49) für die entgaste Flüssigkeit enthaltende Ausgangskappe (42) derart abgeschlossen ist, daß die Enden der Filterkapillaren (44) innerhalb und die Enden (47) der Entlüftungskapillaren (45) außerhalb der Auslaßöffnung (49) für die entgaste Flüssigkeit enden.

11. Vorrichtung nach Anspruch 10, dadurch gekennzeichnet, daß die Enden (47) der Entlüftungskapillaren (45) im Bereich der Dichtmasse (51) zumindest annähernd zum Gehäuse achsparallel angeordnet sind.

12. Vorrichtung nach Anspruch 1, dadurch gekennzeichnet, daß als Abdichtung zwischen Ausgangskappe (31) und Dichtmasse (35), in die die Enden der Filterkapillaren eingebettetsind, im Bereich zwischen den Filterkapillaren (34) und den Entlüftungskapillaren (33) der Ring (32) angeordnet ist.

## Claims

1. A device for the separation of gas bubbles from infusion fluids or fluids of the human body by means of at least one liquid-repelling, gas-permeable, microporous, deaeration component with a housing (30, 53, 31, 42), which has an inlet aperture (37, 48) for the fluid to be degassed, an outlet aperture (36, 49) for the degassed fluid in the form of an outlet cap (31, 42) and at least one outlet aperture (38, 47) for the gas, wherein the deaeration component is arranged at least partially in the region of the housing in which gas bubbles occur, characterised in that the deaeration component comprises at least one deaeration capillary (33, 45), the inner cavity of which is shut off with respect to the fluid, wherein at least one end of each deaeration capillary (33, 45) serves as an outlet aperture (38, 47) for the gas and is embedded in a sealant (51), and each outlet aperture (38, 47) for the gas is connected with a surrounding groove (50) formed by the outlet cap (42), the sealant (51) and the housing (53), or with a surrounding groove (39) formed by the outlet cap (31) and a ring (32) inserted in the housing (30).

2. A device according to claim 1, characterised in that at least one end in each case (38, 47) of the deaeration capillaries (33, 45), arranged in the axial direction of the housing, serves as an outlet aperture for the gas.

3. A device according to claim 1 or 2, characterised in that several deaeration capillaries (33) are distributed on the perimeter on the inside of the housing jacket.

4. A device according to claim 2 or 3, characterised in that several deaeration capillaries arranged parallel to one another in the form of a band are used.

5. A device according to one or more of claims 1 to 4, characterised in that said device additionally contains a wettable, gas-impermeable, microporous filter (34, 44), which divides the housing into a first chamber with the inlet aperture (37, 48) and a second chamber together with the outlet cap (31, 42), wherein the deaeration capillary (capillaries) (33, 45) in the first chamber between filter (34, 44) and housing wall is (are) arranged at least above the filter (34, 44).

6. A device according to claim 5, characterised in that the filter comprises at least one filter capillary (34, 44), and the inner cavity of the filter capillaries forms at least partially the second chamber.

7. A device according to claim 6, characterised in that the deaeration capillary (capillaries) (45) is (are) looped loosely several times round the filter capillary (capillaries) (44) over its (their) length.

8. A device according to claim 6, characterised in that the filter capillaries (34) are arranged in the form of a bundle, on the perimeter of which is (are) arranged the deaeration capillary (capillaries) (33).

9. A device according to claim 6 or 8, characterised in that the filter capillaries and the deaeration capillaries are combined as weft and/or warp threads to form a woven fabric or several woven fabrics.

10. A device according to one or more of claims 6 to 9, characterised in that, in a tubular housing, the filter capillaries (44) and deaeration capillaries (45) are arranged in the form of an elongated U, wherein all the ends (46/47) of the capillaries (44/45) at one end of the housing are embedded in a sealant (51) in such a way that, in the central region of the embedding point, only filter capillaries and in the outer region of the embedding point only deaeration capillaries terminate, the end of the housing provided with the sealant (51) being shut off by an outlet cap (42) containing the outlet aperture (49) for the degassed fluid in such a way that the ends of the filter capillaries (44) terminate inside and the ends (47) of the deaeration capillaries (45) terminate outside the outlet aperture (49) for the degassed fluid.

11. A device according to claim 10, characterised in that the ends (47) of the deaeration capillaries (45) in the region of the sealant (51) are arranged at least approximately parallel to the axis of the housing.

12. A device according to claim 1, characterised in that, as a seal between outlet cap (31) and sealant (35) in which the ends of the filter capillaries are embedded, the ring (32) is arranged in the region between the filter capillaries (34) and the deaeration capillaries (33).

## Revendications

1. Dispositif pour éliminer les bulles de gaz des liquides de perfusion ou des liquides du corps humain à l'aide d'au moins un élément de dégazage microporeux perméable au gaz et hydrophobe, comportant un corps (30, 53, 31, 42) qui est pourvu d'un orifice d'entrée (37, 48) pour le liquide à dégazer, un orifice de sortie (36, 49) pour le liquide débarrassé du gaz, qui est agencé sous la forme d'une coiffe de sortie (31, 42), et au moins un orifice de sortie (38, 47) pour le gaz, l'élément de dégazage étant au moins en partie disposé dans la zone du corps dans laquelle des bulles de gaz apparaissent, caractérisé par le fait que l'élément de dégazage est constitué par au moins un capillaire de dégazage (33, 45) dont la cavité intérieure est fermée vis à vis du liquide, au moins une extrémité de chaque capillaire de dégazage (33, 45) servant d'orifice de sortie (38, 47) pour le gaz et étant noyée dans une masse d'étanchéité (51) et chaque orifice de sortie (38, 47) pour le gaz communiquant avec une gorge (50) périphérique formée par la coiffe de sortie (42), la masse d'étanchéité (51) et le corps (53) ou avec une gorge (39) périphérique formée par la coiffe de sortie (31), et une bague (32) montée dans le corps (53).

2. Dispositif selon la revendication 1, caractérisé par le fait qu'au moins une extrémité (38, 47) des capillaires de dégazage (33, 45) disposée dans la direction axiale du corps sert d'orifice de sortie pour le gaz.

3. Dispositif selon la revendication 1 ou 2, caractérisé par le fait que plusieurs capillaires de dégazage (33) sont répartis sur le pourtour de la paroi du corps, sur la face intérieure de celle-ci.

4. Dispositif selon la revendication 2 ou 3, caractérisé par le fait que l'on utilise plusieurs capillaires de dégazage qui sont disposés parallèlement les uns autres sous la forme d'une petite bande.

5. Dispositif selon l'une ou plusieurs des revendications 1 à 4, caractérisé par le fait que celui-ci comporte en outre un filtre (34, 44) microporeux mouillable, non perméable aux gaz, qui sépare le corps en une première chambre avec l'orifice d'entrée (37, 48) et une deuxième chambre avec la coiffe de sortie (31 , 42), le(s) capillaire(s) (33, 45) de dégazage étant disposé(s) dans la première chambre entre le filtre (34, 44) et la paroi du corps au moins au-dessus du filtre (34, 44).

6. Dispositif selon la revendication 5, caractérisé par le fait que le filtre est constitué par au moins un capillaire de filtration (34, 44) et que la cavité intérieure du ou des capillaire(s) de filtration forme au moins partiellement la deuxième chambre.

7. Dispositif selon la revendication 6, caractérisé par le fait que le(s) capillaire(s) de dégazage (45) est (sont) disposé(s) de manière telle qu'il(s) entoure(nt) de manière lâche plusieurs fois sur sa (leur) longueur le(s) capillaire(s) de filtration (44).

8. Dispositif selon la revendication 6, caractérisé par le fait que les capillaires de filtration (44) sont disposés sous la forme d'un faisceau à la périphérie duquel est (sont) disposé(s) le(s) capillaire(s) de dégazage (33).

9. Dispositif selon la revendication 6 ou 8, caractérisé par le fait que les capillaires de filtration et les capillaires de dégazage sont assemblés en tant que fils de trame et/ou fils de chaîne pour former un tissus ou plusieurs tissus.

10. Dispositif selon l'une ou plusieurs des revendications 6 à 9, caractérisé par le fait que les capillaires de filtration (44) et les capillaires de dégazage (45) sont disposés en forme de U allongé dans un corps tubulaire, toutes les extrémités (46/47) des capillaires (44/45) étant noyées dans une masse d'étanchéité (51) à une extrémité du corps, de manière telle que seuls les capillaires de dégazage débouchent dans la partie centrale de la zone où lesdites extrémités sont noyées, l'extrémité du corps comportant la masse d'étanchéité (51) étant fermée par une coiffe de sortie (42) qui comporte l'orifice de sortie (49) pour le liquide dégazé, de manière telle que les extrémités des capillaires de filtration (44) débouchent dans et les extrémités des capillaires de dégazage (45) débouchent en dehors de l'orifice de sortie (49) de liquide dégazé.

11. Dispositif selon la revendication 10, caractérisé par le fait que, dans la zone de la masse d'étanchéité (51), les extrémités (47) des capillaires de dégazage (45) sont disposées au moins sensiblement parallèlement à l'axe du corps.

12. Dispositif selon la revendication 1, caractérisé par le fait que la bague (32) est disposé en tant que moyen d'étanchéité entre la coiffe de sortie (31) et la masse d'étanchéité (35) dans laquelle sont noyées les extrémités des capillaires de filtration, dans la zone comprise entre les capillaires (34) et les capillaires de dégazage (33).
